# EUROPÄISCHE PATENTSCHRIFT

(11) **EP 0 945 125 B1**
(45) Veröffentlichungstag und Bekanntmachung des Hinweises auf die Patenterteilung: **21.05.2003**
(21) Anmeldenummer: 99102204.7
(22) Anmeldetag: 04.02.1999
(51) Int. Cl.: A61K 7/42

(54) **Photostabile UV-Filter enthaltende kosmetische und pharmazeutische Zubereitungen**
Cosmetic and pharmaceutical compositions containing photostable uv filters
Compositions cosmétiques et pharmaceutiques contenant des filtres UV photostables

(30) Priorität: 16.02.1998 DE 19806241
(43) Veröffentlichungstag der Anmeldung: 29.09.1999
(73) Patentinhaber: BASF AKTIENGESELLSCHAFT, 67056 Ludwigshafen (DE)
(72) Erfinder: Habeck, Thorsten, Dr., 67149 Meckenheim (DE); Krause, Alfred, Dr., 67346 Speyer (DE)

(56) Entgegenhaltungen:
- EP-A- 0 100 651
- EP-A- 0 852 137
- WO-A-98/14423

## Beschreibung

Die Erfindung betrifft die Verwendung von Malonesterderivaten als photostabile UV-Filter in kosmetischen und pharmazeutischen Zubereitungen zum Schutz der menschlichen Epidermis oder menschlicher Haare gegen UV-Strahlung, speziell im Bereich von 320 bis 400 nm.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische und pharmazeutische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschuztmittel zusätzlich eine hoch spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen, toxikologische Unbedenklichkeit sowie geringen Eigengeruch und geringe Eigenfärbung.

Eine weitere Anforderung, der Lichtschutzmittel genügen müssen, ist eine ausreichende Photostabilität. Dies ist aber mit den bisher verfügbaren UV-A absorbierenden Lichtschutzmitteln nicht oder nur unzureichend gewährleistet.

In der französischen Patentschrift Nr. 2 440 933 wird das 4-(1,1-Dimethylethyl)-4'-methoxydibenzoylmethan als UV-A-Filter beschrieben. Es wird vorgeschlagen, diesen speziellen UV-A-Filter, der von der Firma GIVAUDAN unter der Bezeichnung "PAR-SOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß EP-A-0 514 491 die nicht ausreichend photostabilen UV-A-Filter durch den Zusatz von 2-Cyan-3,3-diphenylacrylsäureestern stabilisiert werden, die selbst im UV-B-Bereich als Filter dienen.

Weiterhin wurde gemäß EP-A-0 251 398 schon vorgeschlagen, UV-Aund UV-B-Strahlung absorbierende Chromophore durch ein Bindeglied in einem Molekül zu vereinen. Dies hat den Nachteil, daß einerseits keine freie Kombination von UV-A- und UV-B-Filtern in der kosmetischen Zubereitung mehr möglich ist und daß Schwierigkeiten bei der chemischen Verknüpfung der Chromophore nur bestimmte Kombinationen zulassen.

In der deutschen Patentschrift Nr. 1 087 902 wird die Verwendung von Kondensationsprodukten aus Oxy- bzw. Alkoxybenzaldehyden und CH-aciden Verbindungen als UV-Filter in technischen Anwendungen, beispielsweise in Kunststoffen, nicht aber in kosmetischen oder pharmazeutischen Zubereitungen beschrieben.

Es bestand daher die Aufgabe, Lichtschutzmittel für kosmetische und pharmazeutische Zwecke vorzuschlagen, die im UV-A-Bereich mit hoher Extinktion absorbieren, die photostabil sind, eine geringe Eigenfarbe d.h. eine scharfe Bandenstrukur aufweisen und je nach Substituent in Öl oder Wasser löslich sind.

Diese Aufgabe wurde erfindungsgemäß gelöst durch Verwendung von Malonesterderivaten der Formel I, 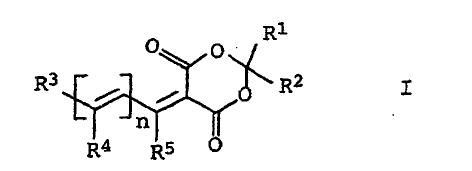 in der die Variablen unabhängig voneinander folgende Bedeutung haben:
- R¹ und R²: Wasserstoff, C₁-C₂₀-Alkyl,
wobei R¹ und R² gemeinsam einen 5- bis 12-Ring bilden können;
- R³: 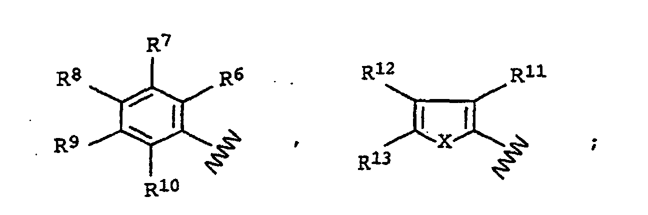
- R⁴: Wasserstoff, C₁-C₂₀-Alkyl, 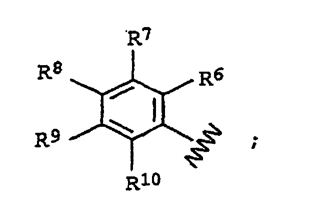
- R⁵: Wasserstoff, C₁-C₂₀-Alkyl, OH, 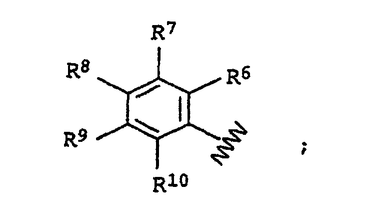
- R⁶ bis R¹³: Wasserstoff, OH, C₁-C₂₀-Alkyl, C₂-C₁₀-Alkenyl, C₃-C₁₀-Cycloalkyl, C₃-C₁₀-Cycloalkenyl, C₁-C₁₂-Alkoxy, C₁-C₂₀-Alkoxycarbonyl, C₁-C₁₂-Alkylamino, C₁-C₁₂-Dialkylamino, Aryl, Heteroaryl, gegebenenfalls substituiert,
wasserlöslich machende Substituenten, ausgewählt aus der Gruppe bestehend aus Carboxylat-, Sulfonat- oder Ammoniumresten;
- X: O, S, NH;
- n: 0, 1
als photostabile UV-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

Als Alkylreste für R¹ und R² sowie für R⁴ bis R¹³ seien verzweigte oder unverzweigte C₁-C₂₀-Alkylketten, bevorzugt Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Die Substituenten R¹ und R² können auch gemeinsam einen 5 bis 12 Kohlenstoffatome enthaltenden Cycloalkylrest bilden. Bevorzugt zu nennen sind Cyclopentyl, Cyclohexyl, Cyclooctyl sowie Cyclododecyl.

Als Alkenylreste für R⁶ bis R¹³ seien verzweigte oder unverzweigte C₂-C₁₀-Alkenylketten, bevorzugt Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, 1-Octenyl oder 2-Octenyl genannt.

Als Cycloalkylreste seien für R⁶ bis R¹³ bevorzugt verzweigte oder unverzweigte C₃-C₁₀-Cycloalkylketten wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclopropyl, 1-Methyl-2-Ethylcyclopropyl, Cyclooctyl, Cyclononyl oder Cyclodecyl genannt.

Als Cycloalkenylreste seien für R⁶ bis R¹³ bevorzugt verzweigte oder unverzweigte, C₃-C₁₀-Cycloalkenylketten mit einer oder mehreren Doppelbindungen wie Cyclopropenyl, Cyclobutenyl, Cyclopentenyl, Cyclopentadienyl, Cyclohexenyl, 1,3-Cyclohexadienyl, 1,4-Cyclohexadienyl, Cycloheptenyl, Cycloheptatrienyl, Cyclooctenyl, 1,5-Cyclooctadienyl, Cyclooctatetraenyl, Cyclononenyl oder Cyclodecenyl genannt.

Die Cycloalkenyl- und Cycloalkylreste können ggf. mit einem oder mehreren, z.B. 1 bis 3 Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein oder 1 bis 3 Heteroatome wie Schwefel, Stickstoff, dessen freie Valenzen durch Wasserstoff oder C₁-C₄-Alkyl abgesättigt sein können oder Sauerstoff im Ring enthalten.

Als Alkoxyreste für R⁶ bis R¹³ kommen solche mit 1 bis 12 C-Atomen, vorzugsweise mit 1 bis 8 C-Atomen in Betracht.

Beispielsweise sind zu nennen:

| | |
|---|---|
| Methoxy- | Ethoxy- |
| Isopropoxy- | n-Propoxy- |
| 1-Methylpropoxy- | n-Butoxy- |
| n-Pentoxy- | 2-Methylpropoxy- |
| 3-Methylbutoxy- | 1,1-Dimethylpropoxy- |
| 2,2-Dimethylpropoxy- | Hexoxy- |
| 1-Methyl-1-ethylpropoxy- | Heptoxy- |
| Octoxy- | 2-Ethylhexoxy- |

Alkoxycarbonylreste für R⁶ bis R¹³ sind z.B Ester, die die oben genannten Alkoxyreste oder Reste von höheren Alkoholen (Fettalkoholen) mit bis zu 20 C-Atomen enthalten.

Als Mono- oder Dialkylaminoreste für R⁶ bis R¹³ kommen solche in Betracht, die Alkylreste mit 1 bis 12 C-Atomen enthalten, wie z.B. Methyl-, n-Propyl-, n-Butyl-, 2-Methylpropyl-, 1,1-Dimethylpropyl-, Hexyl-, Heptyl-, 2-Ethylhexyl-, Isopropyl-, 1-Methylpropyl-, n-Pentyl-, 3-Methylbutyl-, 2,2-Dimethylpropyl-, 1-Methyl-1-ethylpropyl- und Octyl.

Unter Aryl sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein können. Bevorzugt sind ggf. substituiertes Phenyl, Methoxyphenyl und Naphthyl.

Heteroaryl-Reste sind vorteilhafterweise einfache oder kondensierte aromatische Ringsysteme mit einem oder mehreren heteroaromatischen 3- bis 7-gliedrigen Ringen. Als Heteroatome können ein oder mehrere Stickstoff-, Schwefel- und/oder Sauerstoffatome im Ring oder Ringsystem enthalten sein.

Hydrophile d.h. die Wasserlöslichkeit der Verbindungen der Formel I ermöglichende Reste für R¹ und R² sind z.B. Carboxy- und Sulfoxyreste und insbesondere deren Salze mit beliebigen physiologisch verträglichen Kationen, wie die Alkalisalze oder wie die Trialkylammoniumsalze, wie Tri-(hydroxyalkyl)-ammoniumsalze oder die 2-Methylpropan-1-ol-2-ammoniumsalze. Ferner kommen Ammoniumreste, insbesondere Alkylammoniumreste mit beliebigen physiologisch verträglichen Anionen in Betracht.

Bevorzugt sind solche Verbindungen der Formel I, in der
- R¹ und R²: C₁-C₁₂-Alkyl,
wobei R¹ und R² gemeinsam einen 5- bis 12-Ring bilden können;
- R³: 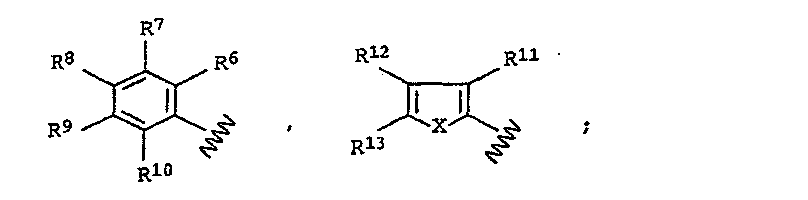
- R⁵: Wasserstoff;
- R⁶ bis R¹³: Wasserstoff, OH, C₁-C₁₂-Alkyl, C₁-C₈-Alkoxy, C₁-C₁₂-Alkoxycarbonyl,
wasserlöslich machende Substituenten, ausgewählt aus der Gruppe bestehend aus Carboxylat-, Sulfonat- oder Ammoniumresten;
- X: O, S, NH;
- n: 0.

Als Alkylreste für R¹ und R² sowie für R⁶ bis R¹³ seien bevorzugt verzweigte oder unverzweigte C₁-C₁₂-Alkylketten, besonders bevorzugt Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl genannt.

Die Substituenten R¹ und R² können auch gemeinsam einen 5 bis 12 Kohlenstoffatome enthaltenden Cycloalkylrest bilden. Bevorzugt zu nennen sind Cyclopentyl, Cyclohexyl und Cyclooctyl.

Als Alkoxyreste für R⁶ bis R¹³ kommen solche mit 1 bis 8 C-Atomen, vorzugsweise mit 1 bis 4 C-Atomen in Betracht.

Beispielsweise sind zu nennen:

| | |
|---|---|
| Methoxy- | Ethoxy- |
| Isopropoxy- | n-Propoxy- |
| 1-Methylpropoxy- | 2-Methylpropoxy- |
| n-Butoxy- | |

Bevorzugte Alkoxycarbonylreste für R⁶ bis R¹³ sind z.B Ester, die die oben genannten Alkoxyreste oder Reste von höheren Alkoholen mit bis zu 12 C-Atomen enthalten.

Besonders bevorzugte Reste für R³ sind u.a. Phenyl, para-substituiertes Phenyl sowie di-(ortho)-substituiertes Phenyl und tri-(meta,para,meta)-substituiertes Phenyl, wobei die Substituenten R⁶ bis R¹⁰ die bereits genannte Bedeutung haben können.

Weiterhin weisen Verbindungen der Formel I besondere photostabile Eigenschaften aus, bei denen die Substituenten R¹ und R² sowie R⁶ bis R¹⁰ in der in den Tabellen 1a und 1b genannten Kombination vorliegen:

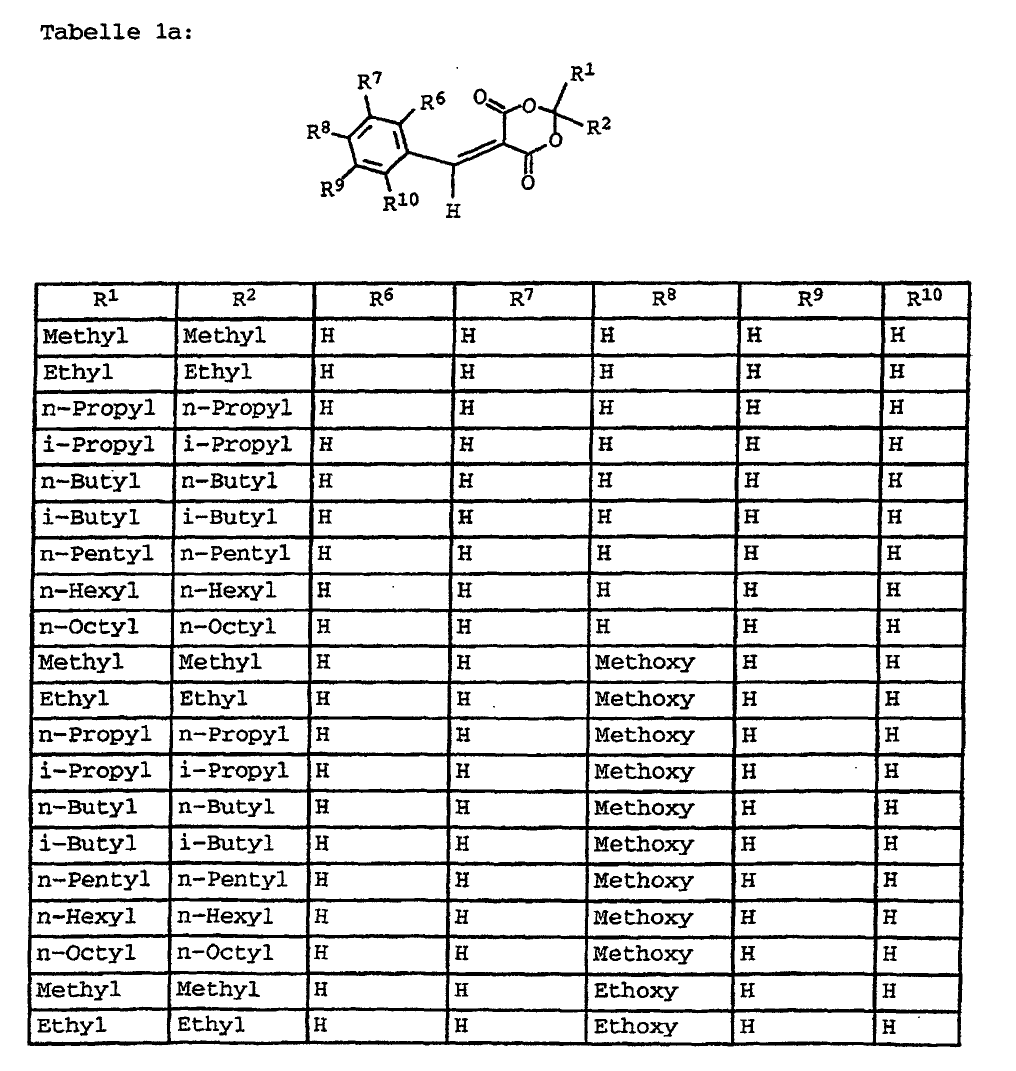

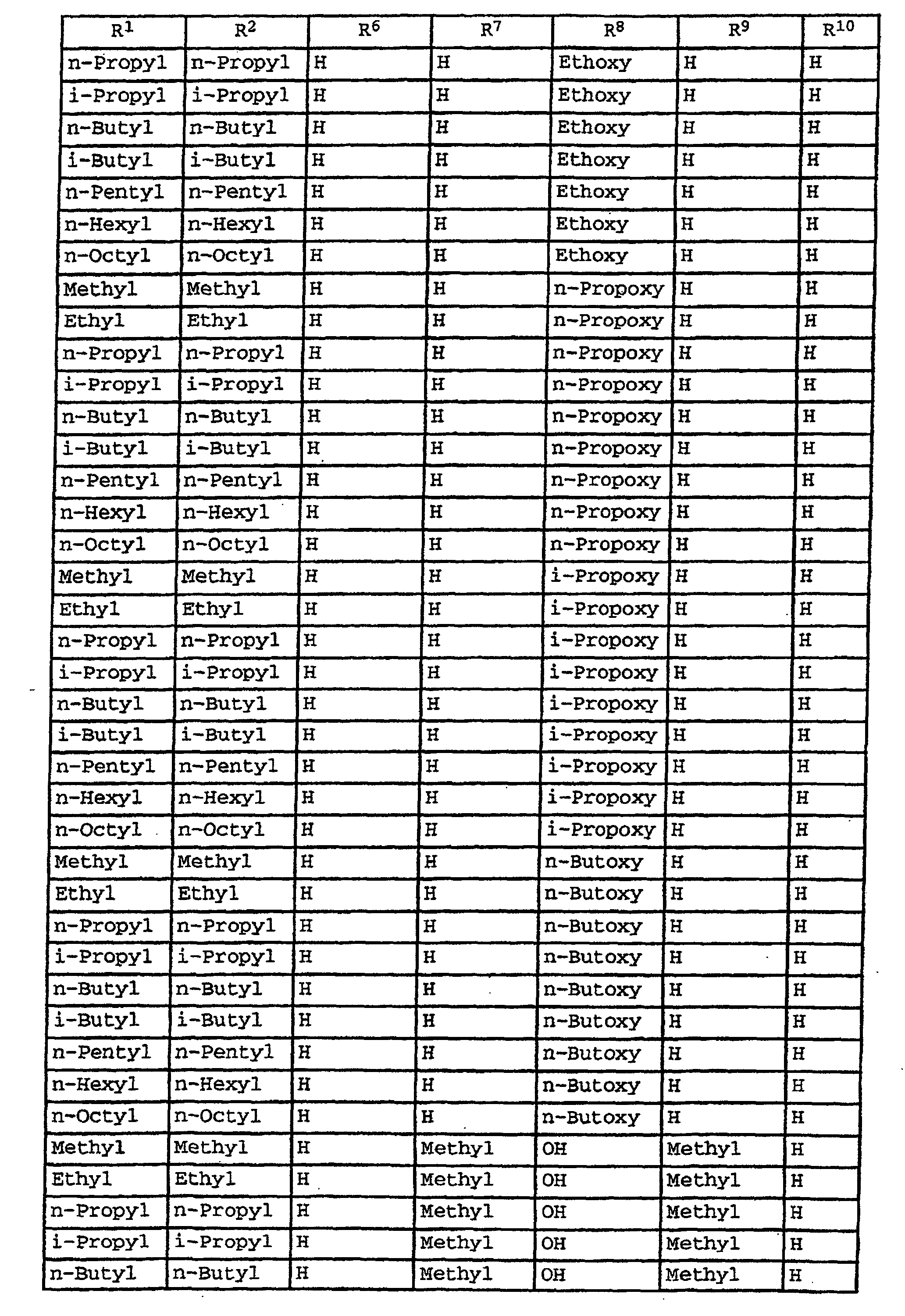

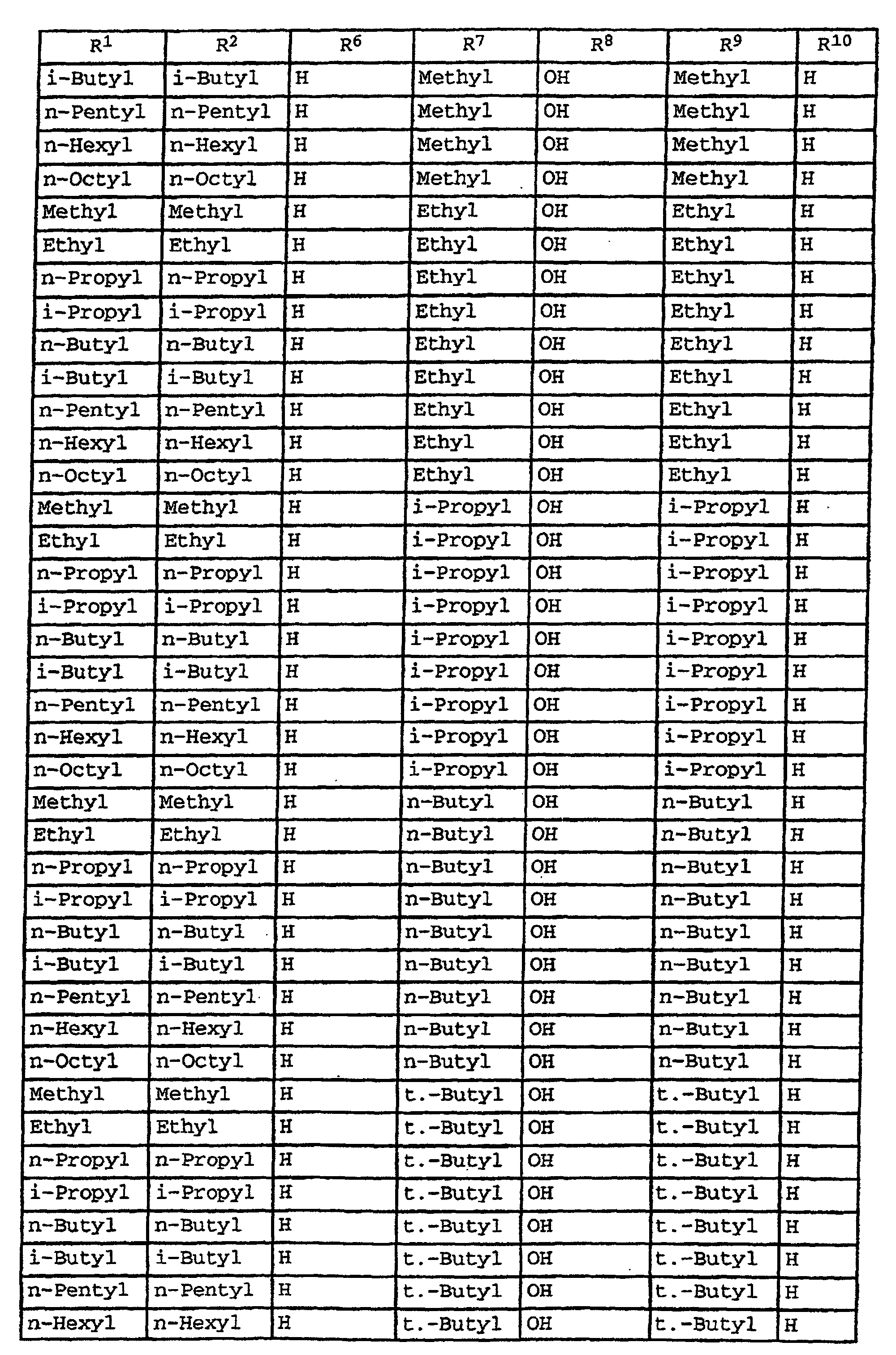

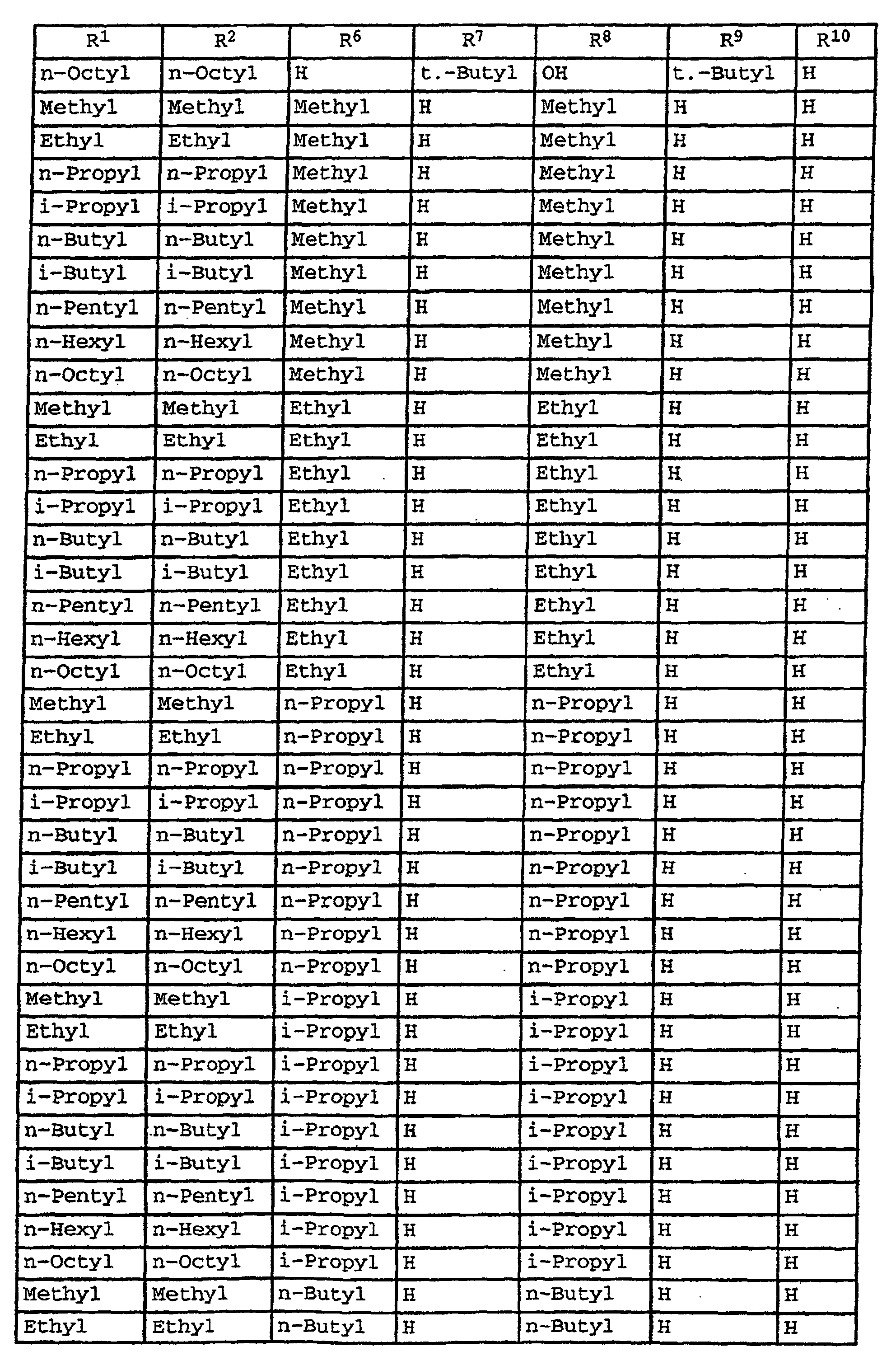

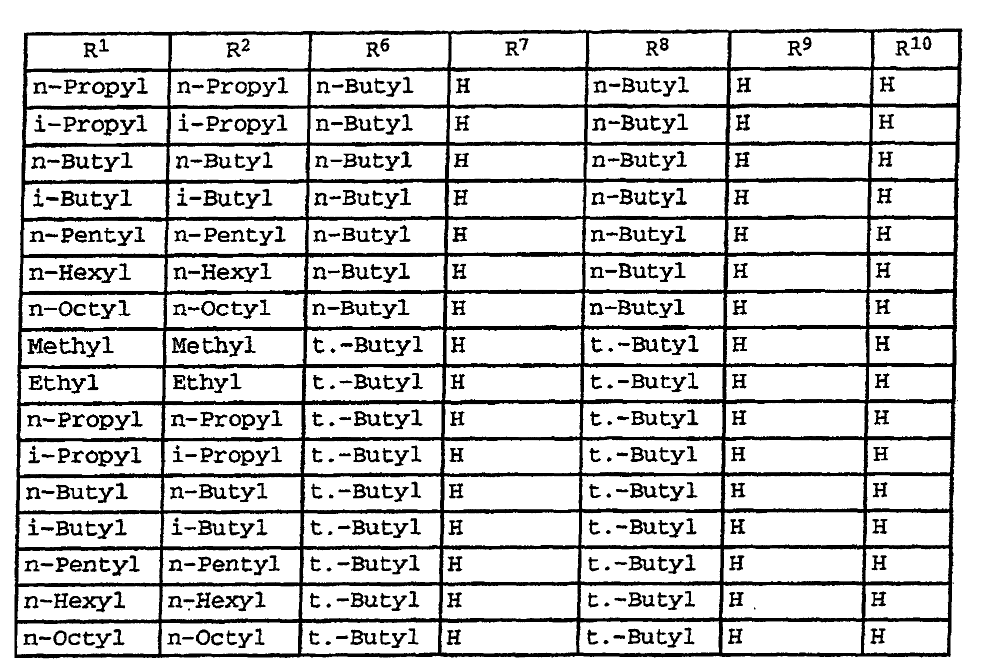

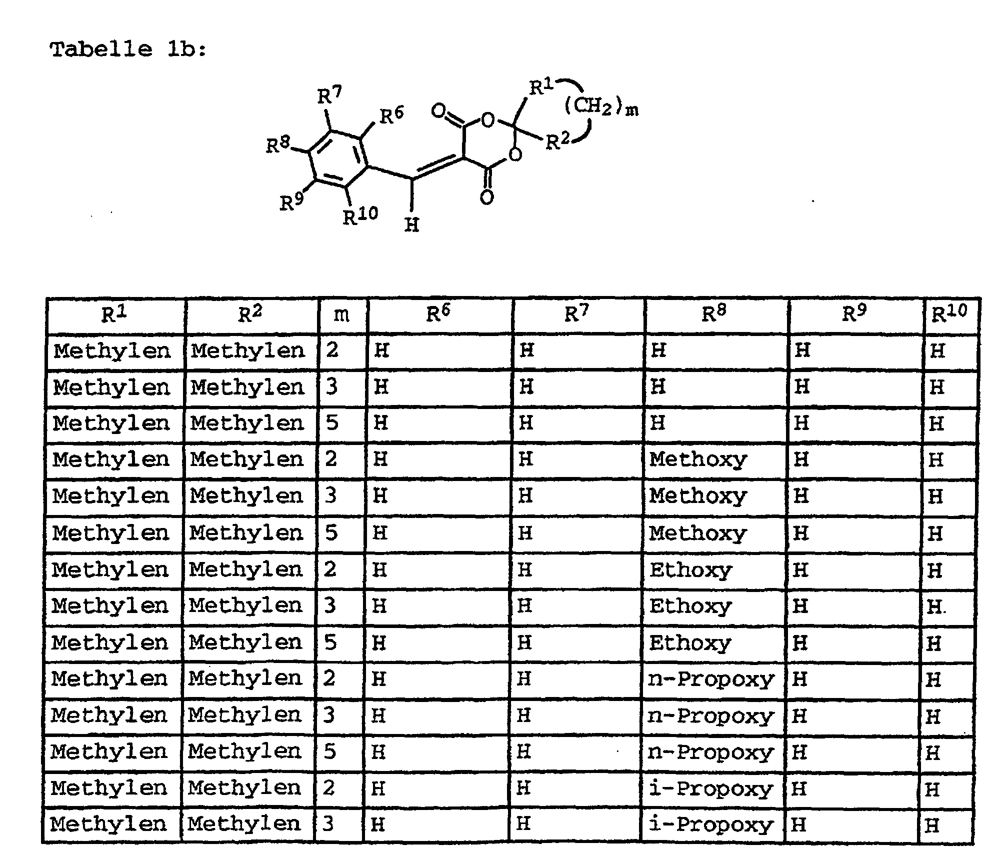

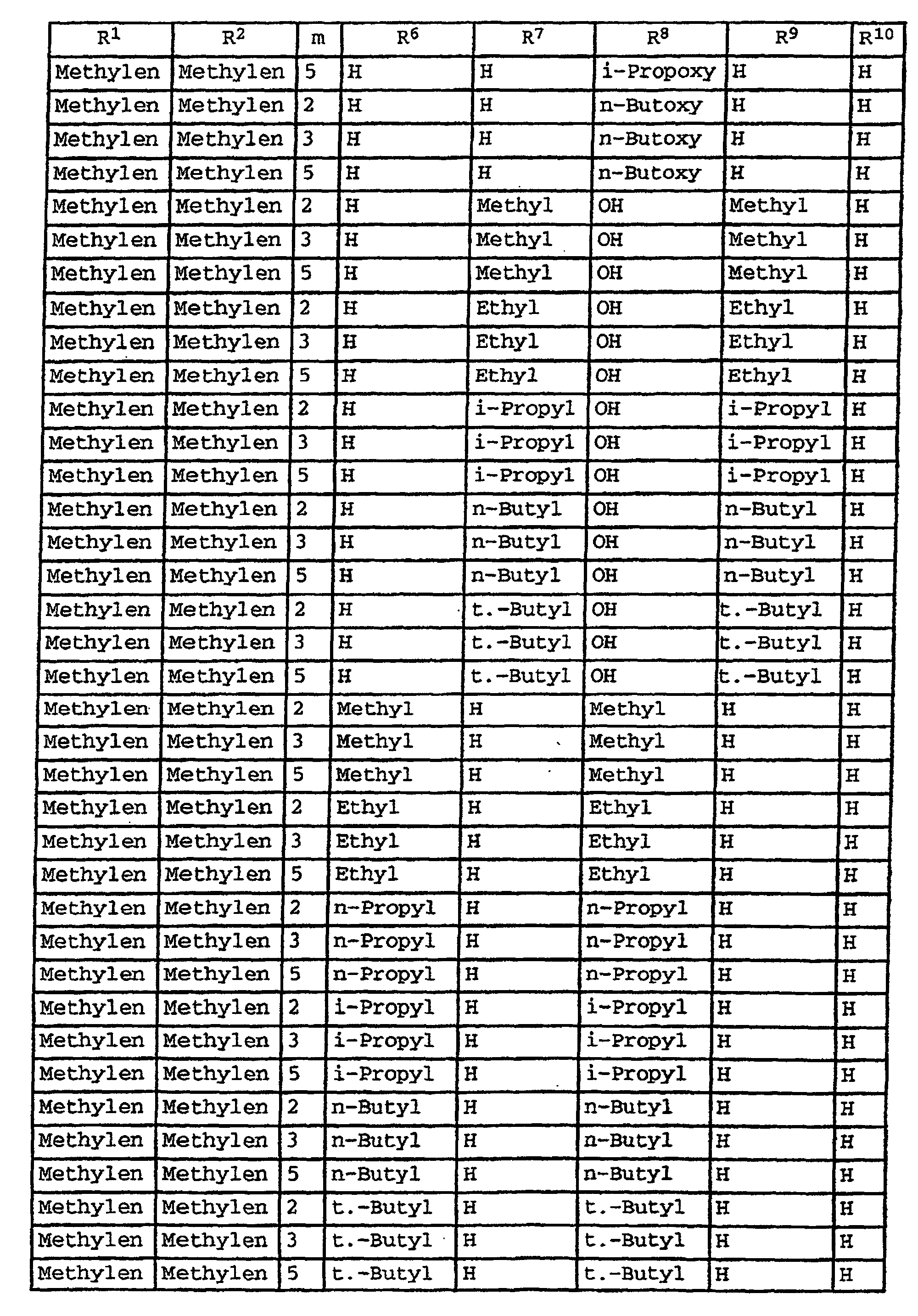

Gegenstand der vorliegenden Erfindung sind weiterhin kosmetische und pharmazeutische Zubereitungen, die 0,1 bis 10 Gew.-%, vorzugsweise 1 bis 7 Gew.-%, bezogen auf die gesamte Menge der kosmetischen und pharmazeutischen Zubereitung, eine oder mehrere der Verbindungen der Formel I zusammen mit an sich für kosmetische und pharmazeutische Zubereitungen bekannten, im UV-B-Bereich absorbierenden Verbindungen als Lichtschutzmittel enthalten, wobei die Verbindungen der Formel I in der Regel in geringerer Menge als die UV-B-absorbierenden Verbindungen eingesetzt werden.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Übliche Ölkomponenten in der Kosmetik sind beispielsweise Paraffinöl; Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magriesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-A-Bereich absorbierenden Substanzen mitverwendet werden, sofern sie im Gesamtsystem der erfindungsgemäß zu verwendenden Kombination aus UV-B und UV-A Filter stabil sind.

Als UV-Filtersubstanzen, die in Kombination mit den erfindungsgemäß zu verwendenden Verbindungen der Formel I angewandt werden, kommen beliebige, u.a. die in Tabelle 2 genannten UV-A- und UV-B-Filtersubstanzen in Betracht.

**Tabelle 2**

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99-5 |
| 20 | 2-Cyano-3,3-diphenylacrylsäure-2'-ethylhexylester | 6197-30-4 |
| 21 | Menthyl-o-aminobenzoate oder: 5-Methyl-2-(1-methylethyl)-2-aminobenzoate | 134-09-8 |
| 22 | Glyceryl p-aminobenzoat oder: 4-Aminobenzoesäure-1-glyceryl-ester | 136-44-7 |
| 23 | 2,2'-Dihydroxy-4-methoxybenzophenon (Dioxybenzone) | 131-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: 3,4-dimethoxy-phenyl-glyoxal-saures Natrium | 4732-70-1 |
| 27 | 3-(4'Sulfo)benzyliden-bornan-2-on und seine Salze | 56039-58-8 |
| 28 | 4-tert.-Butyl-4'-methoxy-dibenzoylmethan | 70356-09-1 |
| 29 | 2,2', 4,4'-Tetrahydroxybenzophenon | 131-55-5 |

Schließlich sind auch mikronisierte Pigmente wie Titandioxid und Zinkoxid zu nennen.

Zum Schutz menschlicher Haare vor UV-Strahlen können die erfindugsgemäßen Lichtschutzmittel der Formel I in Shampoos, Lotionen, Gelen, Haarsprays, Aerosol-Schaumcremes oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Verbindungen zeichnen sich in der Regel durch ein besonders hohes Absorptionsvermögen im Bereich der UV-A-Strahlung mit scharfer Bandenstruktur aus. Weiterhin sind sie gut in kosmetischen Ölen löslich und lassen sich leicht in kosmetische Formulierungen einarbeiten. Die mit den Verbindungen I hergestellten Emulsionen zeichnen sich besonders durch ihre hohe Stabilität, die Verbindungen I selber durch ihre hohe Photostabilität aus, und die mit I hergestellten Zubereitungen durch ihr angenehmes Hautgefühl aus.

Die UV-Filterwirkung der erfindungsgemäßen Verbindungen der Formel I kann auch zur Stabilisierung von Wirk- und Hilfsstoffen in kosmetischen und pharmazeutischen Formulierungen ausgenutzt werden.

Die für die Verwendung als Lichtschutzmittel in kosmetischen und pharmazeutischen Zubereitungen beanspruchten Verbindungen der Formel I werden durch Kondensation einer aromatischen Carbonylverbindung der Formel II mit cyclischen Malonestern der Formel III erhalten, wobei die Reste R¹ bis R⁵ die eingangs genannten Bedeutungen haben sollen:

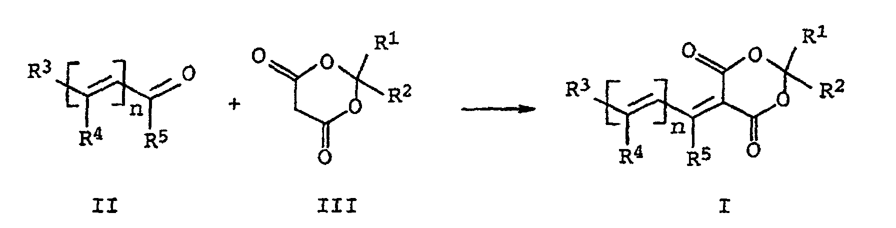

Die nachfolgenden Beispiele sollen die vorliegende Erfindung verdeutlichen, ohne sie einzuschränken.

### Beispiel 1

### Herstellung von Verbindung 1 der Tabelle 3a

0.1 mol Benzaldehyd und 0.1 mol Meldrumsäure wurden in 100 ml Methanol gelöst, mit je 1 ml Piperidin und Eisessig versetzt und 5 h unter Rückfluß erhitzt. Anschließend wurde mit Wasser verdünnt und mit Dichlormethan extrahiert. Die organische Phase wurde mit Aktivkohle aufgerührt. Nach Abfiltrieren der organischen Phase wurde über Natriumsulfat getrocknet und im Vakuum eingeengt. Man erhielt 19 g (81 % d. Th.) der Verbindung 1 der Tabelle 3a als hellgelbe Kristalle. Reinheit: > 99 % (GC)

Die übrigen Verbindungen 2 bis 12 der Tabellen 3a und 3b wurden analog Beispiel 1 aus den entsprechend substituierten aromatischen Aldehyden und den jeweiligen Malonestern erhalten.

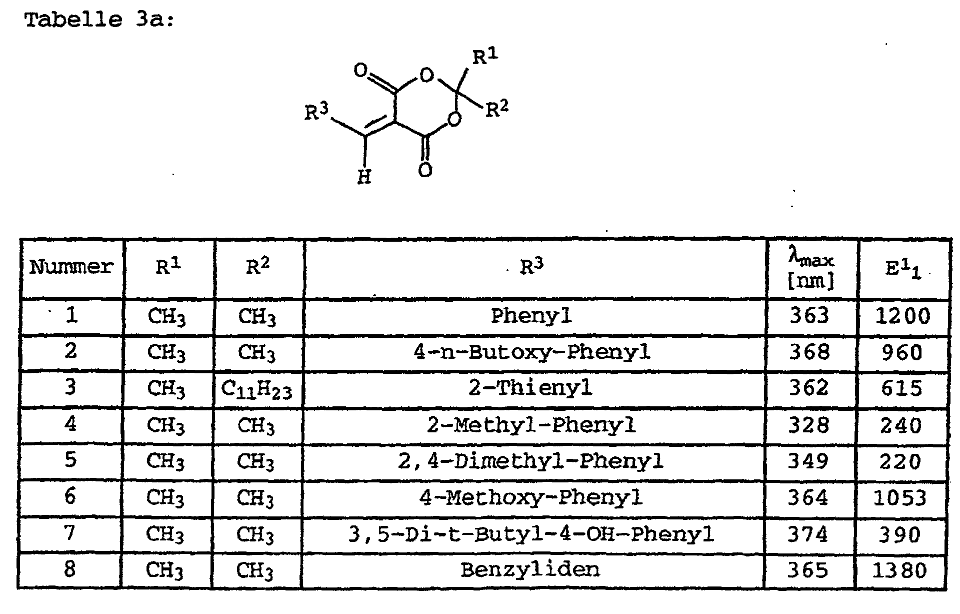

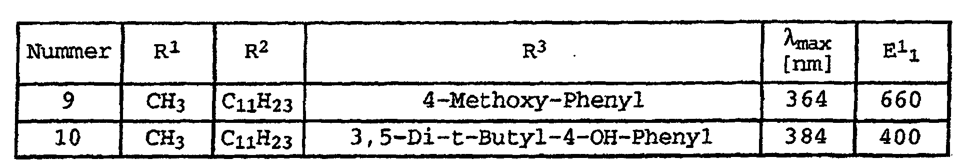

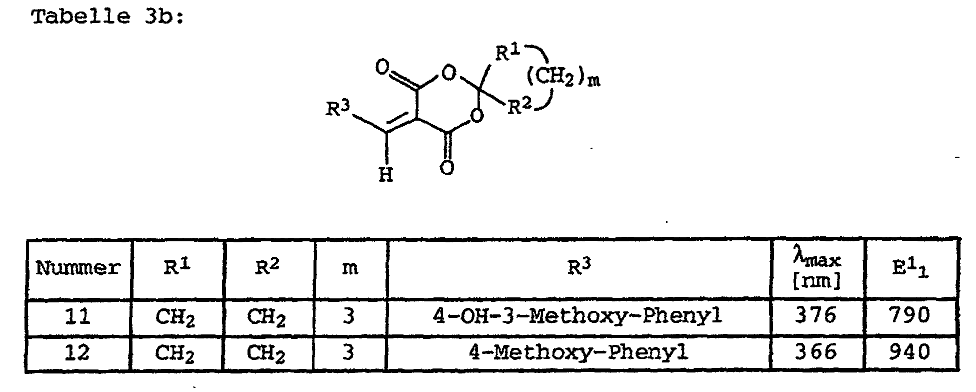

### Beispiel 2

### Standardisierte Methode zur Bestimmung der Photostabilität (Suntest)

Eine 5 Gew.-%ige alkoholische Lösung des zu prüfenden Lichtschutzmittels wird mittels einer Eppendorfpipette (20 µl) auf die Auffräsung eines Glasplättchens aufgetragen. Durch die Anwesenheit des Alkohols verteilt sich die Lösung gleichmäßig auf der aufgerauten Glasoberfläche. Die aufgetragene Menge entspricht der Menge an Lichtschutzmittel, die in Sonnencremes zur Erreichung eines mittleren Lichtschutzfaktors benötigt wird. Bei der Prüfung werden jeweils 4 Glasplättchen bestrahlt. Die Abdampf zeit und die Bestrahlung betragen je 30 Minuten. Die Glasplättchen werden während des Bestrahlens durch eine Wasserkühlung, die sich am Boden des Suntestgeräte befindet, leicht gekühlt. Die Temperatur innerhalb des Suntest Gerätes beträgt während der Bestrahlung 40°C. Nachdem die Proben bestrahlt worden sind, werden sie mit Ethanol in einen dunklen 50 ml Meßkolben gewaschen und mit dem Photometer vermessen. Die Blindproben werden ebenso auf Glasplättchen aufgetragen und 30 Minuten bei Raumtemperatur abgedampft. Wie die anderen Proben werden sie mit Ethanol abgewaschen und auf 100 ml verdünnt und vermessen.

Die nach der obigen Methode untersuchten, erfindungsgemäßen Verbindungen der Formel I zeigten eine Photostabilität von > 90 %.

Allgemeine Herstellvorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

### Zubereitungen

### Beispiel 3

Zusammensetzung für die Lippenpflege

| Massengehalt | |
|---|---|
| Gew.-% | |
| ad 100 | Eucerinum anhydricum |
| 10,00 | Glycerin |
| 10,00 | Titanium Dioxid |
| 5,00 | Verbindung Nr. 1 der Tabelle 3a |
| 8,00 | Octyl Methoxycinnamat |
| 5,00 | Zink Oxid |
| 4,00 | Castoröl |
| 4,00 | Pentaerythrityl Stearat/Caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 4

Zusammensetzung für Sunblocker mit Mikropigmenten

| Massengehalt | |
|---|---|
| Gew.-% | |
| ad 100 | Wasser |
| 10,00 | Octyl Methoxycinnamat |
| 6,00 | PEG-7-Hydrogenated Castor Öl |
| 6,00 | Titanium Dioxid |
| 5,00 | Verbindung Nr. 1 der Tabelle 3a |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 5

Fettfreies Gel

| Massengehalt | |
|---|---|
| Gew.-% | |
| ad 100 | Wasser |
| 8,00 | Octyl Methoxycinnamat |
| 7,00 | Titanium Dioxid |
| 5,00 | Verbindung Nr. 1 der Tabelle 3a |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 6

Sonnencreme (LSF 20)

| Massengehalt | |
|---|---|
| Gew.-% | |
| ad 100 | Wasser |
| 8,00 | Octyl Methoxycinnamat |
| 8,00 | Titanium Dioxid |
| 6,00 | PEG-7-Hydrogenated Castor Öl |
| 5,00 | Verbindung Nr. 1 der Tabelle 3a |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 7

Sonnencreme wasserfest

| Massengehalt | |
|---|---|
| Gew.-% | |
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindung Nr. 1 der Tabelle 3a |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 8

Sonnenmilch (LSF 6)

| Massengehalt | |
|---|---|
| Gew.-% | |
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Verbindung Nr. 1 der Tabelle 3a |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

## Patentansprüche

1. Verwendung von Malonesterderivaten der Formel I, 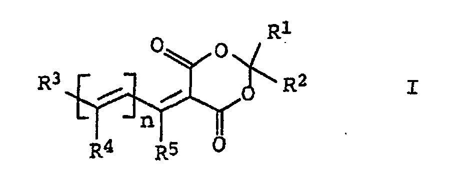 in der die Variablen unabhängig voneinander folgende Bedeutung haben:
R¹ und R² Wasserstoff, C₁-C₂₀-Alkyl,
wobei R¹ und R² gemeinsam einen 5- bis 12-Ring bilden können;
R³ 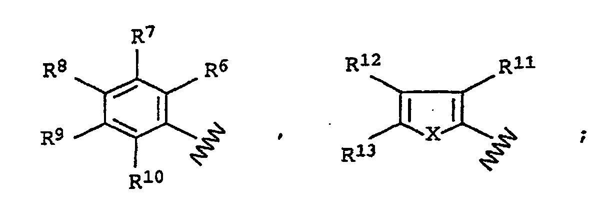
R⁴ Wasserstoff, C₁-C₂₀-Alkyl, 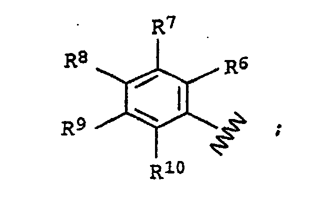
R⁵ Wasserstoff, C₁-C₂₀-Alkyl, OH, 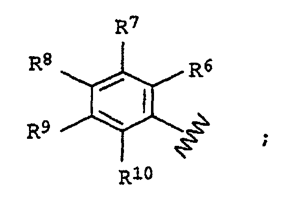
R⁶ bis R¹³ Wasserstoff, OH, C₁-C₂₀-Alkyl, C₂-C₁₀-Alkenyl, C₃-C₁₀-Cycloalkyl, C₃-C₁₀-Cycloalkenyl, C₁-C₁₂-Alkoxy, C₁-C₂₀-Alkoxycarbonyl, C₁-C₁₂-Alkylamino, C₁-C₁₂-Dialkylamino, Aryl, Heteroaryl, gegebenenfalls substituiert, wasserlöslich machende Substituenten, ausgewählt aus der Gruppe bestehend aus Carboxylat-, Sulfonat- oder Ammoniumresten;
X O, S, NH;
n 0, 1
als photostabile UV-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

2. Verwendung von Verbindungen der Formel I nach Anspruch 1 als photostabile UV-A-Filter.

3. Verwendung von Verbindungen der Formel I nach den Ansprüchen 1 und 2 als UV-Stabilisator in kosmetischen und pharmazeutischen Formulierungen.

4. Verwendung von Verbindungen der Formel I nach den Ansprüchen 1 bis 3, wobei die Substituenten unabhängig voneinander folgende Bedeutung haben:
R¹ und R² C₁-C₁₂-Alkyl
wobei R¹ und R² gemeinsam einen 5- bis 12-Ring bilden können;
R³ 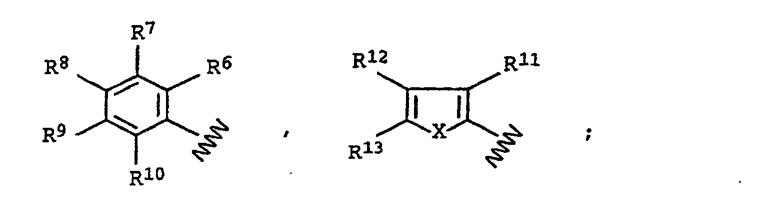
R⁵ Wasserstoff;
R⁶ bis R¹³ Wasserstoff, OH, C₁-C₁₂-Alkyl, C₁-C₁₂-Alkoxy, C₁-C₁₂-Alkoxycarbonyl,
wasserlöslich machende Substituenten, ausgewählt aus der Gruppe bestehend aus Carboxylat-, Sulfonat- oder Ammoniumresten;
X O, S, NH;
n 0.

5. Lichtschutzmittel enthaltende kosmetische und pharmazeutische Zubereitungen zum Schutz der menschlichen Epidermis oder menschlichen Haare gegen UV-Licht im Bereich von 280 bis 400 nm, **dadurch gekennzeichnet, daß** sie in einem kosmetisch und pharmazeutisch geeigneten Träger, allein oder zusammen mit für kosmetische und pharmazeutische Zubereitungen bekannten im UV-Bereich absorbierenden Verbindungen, als photostabile UV-Filter wirksame Mengen von Verbindungen der Formel I 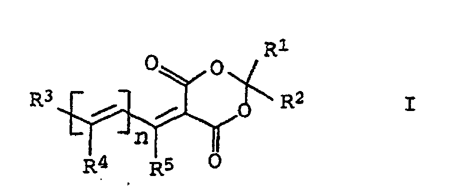 enthalten, in der die Variablen die Bedeutung gemäß Anspruch 1 haben.

6. Lichtschutzmittel enthaltende kosmetische und pharmazeutische Zubereitungen nach Anspruch 5, enthaltend als UV-A-Filter Verbindungen der Formel I, in der die Variablen die Bedeutung gemäß Anspruch 4 haben.

## Claims

1. The use of malonic ester derivatives of the formula I 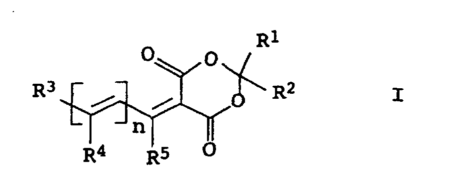 in which the variables have the following meanings, independently of one another:
R¹ and R² hydrogen, C₁-C₂₀-alkyl,
where R¹ and R² may together form a 5- to 12-membered ring;
R³ 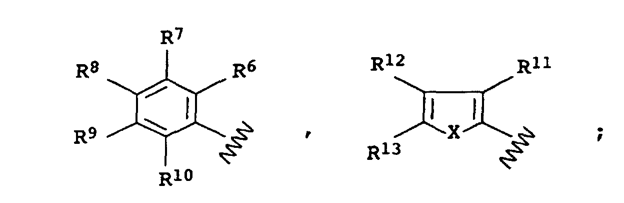
R⁴ hydrogen, C₁-C₂₀-alkyl, 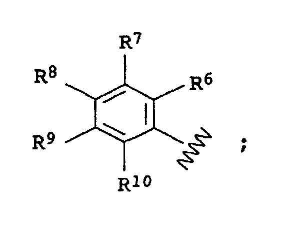
R⁵ hydrogen, C₁-C₂₀-alkyl, OH, 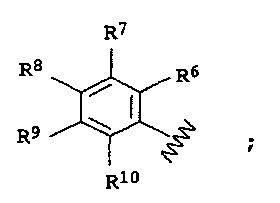
R⁶ to R¹³ hydrogen, OH, C₁-C₂₀-alkyl, C₂-C₁₀-alkenyl, C₃-C₁₀-cycloalkyl, C₃-C₁₀-cycloalkenyl, C₁-C₁₂-alkoxy, C₁-C₂₀-alkoxycarbonyl, C₁-C₁₂-alkylamino, C₁-C₁₂-dialkylamino, aryl, hetaryl, unsubstituted or substituted, substances which confer solubility in water and are selected from the group consisting of carboxylate, sulfonate or ammonium residues;
X O, S, NH;
n 0, 1
as photostable UV-filter in cosmetic and pharmaceutical preparations for protecting the human skin or human hair from the sun's rays, alone or together with compounds which absorb in the UV region and are known per se for cosmetic and pharmaceutical preparations.

2. The use of compounds of the formula I as claimed in claim 1 as photostable UV-A filters.

3. The use of compounds of the formula I as claimed in claims 1 and 2 as UV stabilizer in cosmetic and pharmaceutical formulations.

4. The use of compounds of the formula I as claimed in any of claims 1 to 3, where the substituents have the following meanings independently of one another:
R¹ and R² C₁-C₁₂-alkyl
where R¹ and R² may together form a 5- to 12-membered ring;
R³ 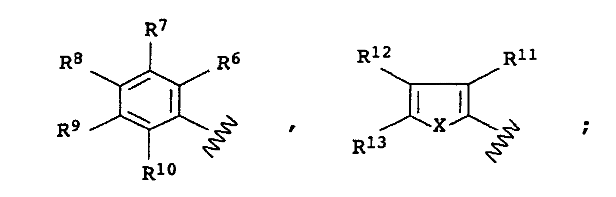
R⁵ hydrogen;
R⁶ to R¹³ hydrogen, OH, C₁-C₁₂-alkyl, C₁-C₈-alkoxy, C₁-C₁₂-alkoxycarbonyl, substituents which confer solubility in water and are selected from the group consisting of carboxylate, sulfonate or ammonium residues;
X O, S, NH;
n 0.

5. A sunscreen-containing cosmetic or pharmaceutical preparation for protecting the human epidermis or human hair from UV light in the region from 280 to 400 nm, which comprises, in a cosmetically or pharmaceutically carrier, alone or together with compounds absorbing in the UV region and known for cosmetic or pharmaceutical preparations, as photostable UV filters effective amounts of compounds of the formula I 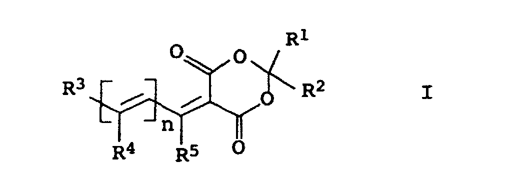 where the variables have the meanings stated in claim 1.

6. A sunscreen-containing cosmetic or pharmaceutical preparation as claimed in claim 5, comprising as UV-A filters compounds of the formula I where the variables have the meanings stated in claim 4.

## Revendications

1. Utilisation de dérivés d'esters maloniques de formule I, 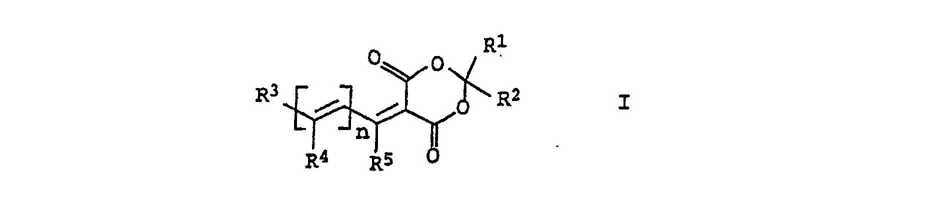 dans laquelle les variables ont, indépendamment l'une de l'autre, la signification suivante:
R¹ et R² hydrogène, alkyle en C₁-C₂₀,
tandis que R¹ et R² peuvent former ensemble un cycle à 5 à 12 chaînons;
R³ 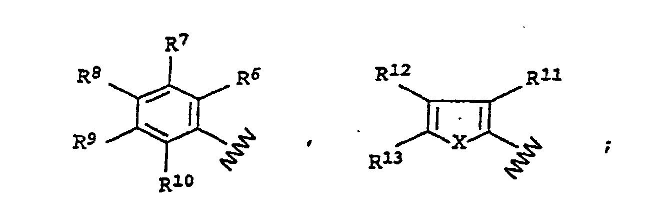
R⁴ hydrogène, alkyle en C₁-C₂₀, 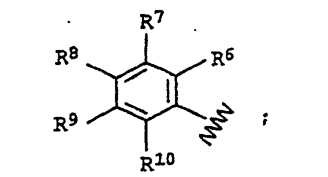
R₅ hydrogène, alkyle en C₁-C₂₀, OH,
R⁶ à R¹³ hydrogène, OH, alkyle en C₁-C₂₀, alcényle en C₂-C₁₀, cycloalkyle en C₃-C₁₀, cycloalcényle en C₃-C₁₀, alcoxy en C₁-C₁₂, alcoxy(C₁-C₂₀)carbonyle, alkylamino en C₁-C₁₂, dialkylamino en C₁-C₁₂, aryle, hétéroaryle, des substituants hydrosolubilisants, éventuellement substitués, choisis dans le groupe consistant en des restes carboxylate, sulfonate ou ammonium;
X O, S, NH;
n 0,1
comme filtres UV photostables dans des compositions cosmétiques et pharmaceutiques pour la protection de la peau humaine ou des cheveux humains contre les rayons solaires, seuls ou conjointement avec des composés absorbants dans le domaine UV, connus pour des compositions cosmétiques et pharmaceutiques.

2. Utilisation de composés de formule I selon la revendication 1 comme filtres UV-A photostables.

3. Utilisation de composés de formule I selon les revendications 1 et 2 comme stabilisant vis-à-vis des UV dans des compositions cosmétiques et pharmaceutiques.

4. Utilisation de composés de formule I selon les revendications 1 à 3, dans laquelle les substituants ont, indépendamment l'un de l'autre, la signification suivante:
R¹ et R² alkyle en C₁-C₁₂
tandis que R¹ et R² peuvent former ensemble un cycle à 5 à 12 chaînons;
R³ 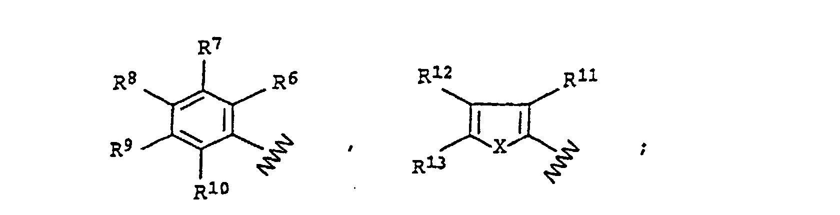
R⁵ hydrogène;
R⁶ à R¹³ hydrogène, OH, alkyle en C₁-C₁₂, alcoxy en C₁-C₁₂, alcoxy(C₁-C₁₂)carbonyle, des substituants hydrosolubilisants, choisis dans le groupe consistant en des restes carboxylate, sulfonate ou ammonium;
X O, S, NH;
n 0.

5. Compositions cosmétiques et pharmaceutiques contenant des agents de protection contre la lumière pour la protection de l'épiderme humain ou des cheveux humains contre la lumière UV dans la gamme de 280 à 400 nm, **caractérisées par le fait qu'**elles contiennent, dans un support cosmétiquement ou pharmaceutiquement approprié, seuls ou en combinaison avec des composés absorbant dans le domaine UV connus pour des préparations cosmétiques et pharmaceutiques, en tant que filtre UV photostable, des quantités efficaces de composés de formule I 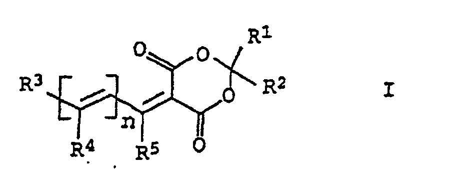 où les variables ont la signification selon la revendication 1.

6. Compositions cosmétiques et pharmaceutiques contenant des agents de protection contre la lumière selon la revendication 5, contenant comme filtre UV-A des composés de formule I, dans laquelle les variables ont la signification selon la revendication 4.
